# EUROPÄISCHE PATENTSCHRIFT

(11) **EP 0 427 902 B1**
(45) Veröffentlichungstag und Bekanntmachung des Hinweises auf die Patenterteilung: **02.03.1994**
(21) Anmeldenummer: 89810930.1
(22) Anmeldetag: 08.12.1989
(51) Int. Cl.: A61F 2/36

(54) **Geradschaft für eine Hüftgelenksprothese**
Rectilinear stam for hip joint prosthesis
Tige rectiligne pour prothèse d'articulation de la hanche

(30) Priorität: 16.11.1989 CH 4131/89
(43) Veröffentlichungstag der Anmeldung: 22.05.1991
(73) Patentinhaber: SULZER Medizinaltechnik AG, 8404 Winterthur (CH)
(72) Erfinder: Zweymüller, Karl, Prof. Dr. med., A-1090 Wien (AT); Koch, Rudolf, CH-8267 Berlingen (CH)
(74) Vertreter: Hammer, Bruno, Dr.

(56) Entgegenhaltungen:
- EP-A- 0 131 178
- EP-A- 0 289 922
- DE-A- 2 839 092
- DE-A- 3 811 207
- FR-A- 2 502 939
- FR-A- 2 539 295

## Beschreibung

Die Erfindung betrifft einen Geradschaft für eine Hüftgelenkprothese zur Verankerung im Femur gemäß des Merkmales des Oberbegriffs des Anspruchs 1.

Zumindest im distalen Bereich werden Operationshohlräume zur Aufnahme von Verankerungsschäften von Femurkopfprothesen sehr häufig als mindestens nahezu kreisrunde, konische Bohrungen ausgeführt. In der Praxis hat sich dabei gezeigt, dass in derartige, künstlich geschaffene Hohlräume mit Vorteil Schäfte mit eckigem Querschnitt eingesetzt werden; denn bei Schäften, die sich vor allem mit ihren Ecken auf dem Knochen abstützen, verbleiben zwischen den Flächen des Schaftes und den Knochenwänden freie Räume, in die vorzugsweise bei nach lateral und medial gerichteten Flächen Knochensubstanz einwächst, während in diesen Räumen vor nach anterior und posterior weisenden Flächen eine Revaskularisierung des Knochengewebes erfolgt, die die Ernährung des lebenden Knochengewebes verbessert.

Es sind daher Schaftkonstruktionen der eingangs beschriebenen Art bekannt (DE-A-38 11 207); bei diesen bekannten Schäften ist der distale Bereich als gerader Stab mit konstantem dreieckähnlichem Querschnitt ausgeführt, während im proximalen sich stark erweiternden Bereich Sägezahnförmige Anlageflächen gezeigt sind, die sich verankern können, wenn der stabförmige distale Teil ungehindert in den Knochenhohlraum abgesenkt werden kann. Da der distale Teil wenig zur Primärverankerung beiträgt, wird diese Lösung auch mit Verwendung von Knochenzement vorgeschlagen. Eine andere Möglichkeit besteht darin, das distale Ende als schwachen, selbsthemmenden Konus auszuführen wie er in der CH-PS 642 252 beschrieben ist. Die Praxis hat nun gezeigt, dass beim Einschlagen oder Einpressen derartiger Schäfte in dem umgebenden kortikalen Knochengewebe Ringspannungen entstehen, die in Umfangsrichtung sehr stark variieren.

Die Erfahrungen mit den letztgenannten Schäften, deren nach anterior und nach posterior weisende Blattseiten mit rillenartigen Vertiefungen versehen sind, haben weiterhin gezeigt, dass Knochengewebe in solche Vertiefungen nur schlecht einwächst und, dass das diese Vertiefungen ausfüllende Gewebe im allgemeinen wenig "stabiles" Bindegewebe ist.

Aufgabe der Erfindung ist es daher, beim Verankern der proximalen Schaftflächen für die Primärverankerung der distalen Schaftflächen eine möglichst gleichmässige Verteilung der genannten Ringspannungen in Umfangsrichtung zu erreichen und gleichzeitig die Schaftoberfläche so zu gestalten, dass das an die Prothese anwachsende Gewebe möglichst weitgehend aus spongiösen Knochengewebe besteht.

Diese zweifache Aufgabe wird nach der Erfindung durch die Merkmale von Anspruch 1 gelöst.

Durch die neue Schaftform entstehen zwischen der im allgemeinen zylindrischen Bohrung des Operationshohlraumes und den Seiten des Quadratquerschnittes, wie bei rechteckigen Schäften, Zwischenräume, in denen sich spongioses Knochengewebe bildet bzw. eine Revaskularisierung des Knochens stattfindet. Dabei hat es sich als vorteilhaft erwiesen, wenn die Seiten des Quadrates parallel und senkrecht zur Ebene von Längsmittelachse und Prothesenhalsachse verlaufen. Bei dieser Ausrichtung der Quadratseiten hat sich nämlich ergeben, dass in den Zwischenräume, die nach anterior und posterior gerichtet sind, die Revaskularisierung des Knochengewebes erfolgt, während sich in den nach lateral und medial gelegenen Zwischenräumen neue Spongiosa bildet.

Bei der neuen Schaftform gewährleisten die Zähne in den Ecken des quadratischen Querschnittes weiterhin eine stabile Abstützung auf dem kortikalen Knochen, wobei die Ausrichtung der sägezahnartigen Zähne mit ihren steilen Flanken nach proximal die im operativ geschaffenen Hohlraum verbliebene Spongiosa verdichtet, statt "zerschneidet". Weiterhin dienen die nach proximal weisenden steilen Zahnflanken dazu, die lateral im allgemeinen als Zug auftretende Belastung in eine Druckbelastung für den Knochen "umzuwandeln". Um dabei die "Angriffsfläche" für diese Druckbelastung zu vergrössern, ist es vorteilhaft, wenn die Zahntiefe der lateral der Mittelebene gelegenen Zähne grösser ist als diejenige der medial davon liegenden.

Die Herstellung des neuen Schaftes wird erheblich vereinfacht, wenn die Zähne in einzelnen Zahnkränzen angeordnet sind. Die Zähne können dann durch eine spanabhebende Bearbeitung eines rotierenden quadratischen Rohlings geschaffen werden, wobei sie entweder unmittelbar aufeinander folgen oder durch Zwischenräume, in denen der quadratische Querschnitt erhalten bleibt, voneinander getrennt sein können.

Soll bei der Implantation des Schaftes neben der erwähnten Verdrängung des spongiösen Knochens im medialen Bereichs des Knochens eine erhöhte Schneidwirkung der Zähne erreicht weden, so können die steilen Zahnflanken der medial der Mittelebene gelegenen Zähne nach distal gerichtet sein.

Im folgenden wird die Erfindung anhand eines Ausführungsbeispiels im Zusammenhang mit der Zeichnung näher erläutert.
- Fig. 1: zeigt den neuen Schaft in einer schematischen Ansicht von anterior oder posterior her gesehen;
- Fig. 2: ist eine Ansicht von Fig. 1 von links, während
- Fig. 3: den Schnitt III-III von Fig. 1 wiedergibt; die
- Fig. 4 - 7: stellen Varianten in der Anordnung der Zähne an den Ecken des Quadrats dar.

Der Schaft 1 einer Femurkopfprothese erweitert sich von seinem distalen Ende 2 nach allen Seiten konisch, wobei der Konus symmetrisch zu einer Längsmittelachse 3 ausgebildet ist. Medial geht der Konus in einen Bogen über, der sich in einem Prothesenhals 4 fortsetzt; auf diesen ist ein sich nach aussen konisch verjüngender Zapfen 5 aufgesetzt, der den nicht gezeigten kugelförmigen Gelenkkopf aufnimmt. Die Prothesenhalsachse 6 schneidet die Längsmittelachse 3 des Schaftes unter einem Winkel, der im wesentlichen dem Winkel zwischen dem Schenkelhals und der Femurachse eines natürlichen Hüftgelenkes entspricht.

Lateral geht der Konus in einen Trochanter-Flügel 7 über, der in einem Bogen zum horizontalen proximalen Ende 8 des Schaftes verläuft, das ebenfalls in dem Prothesenhals 4 endet.

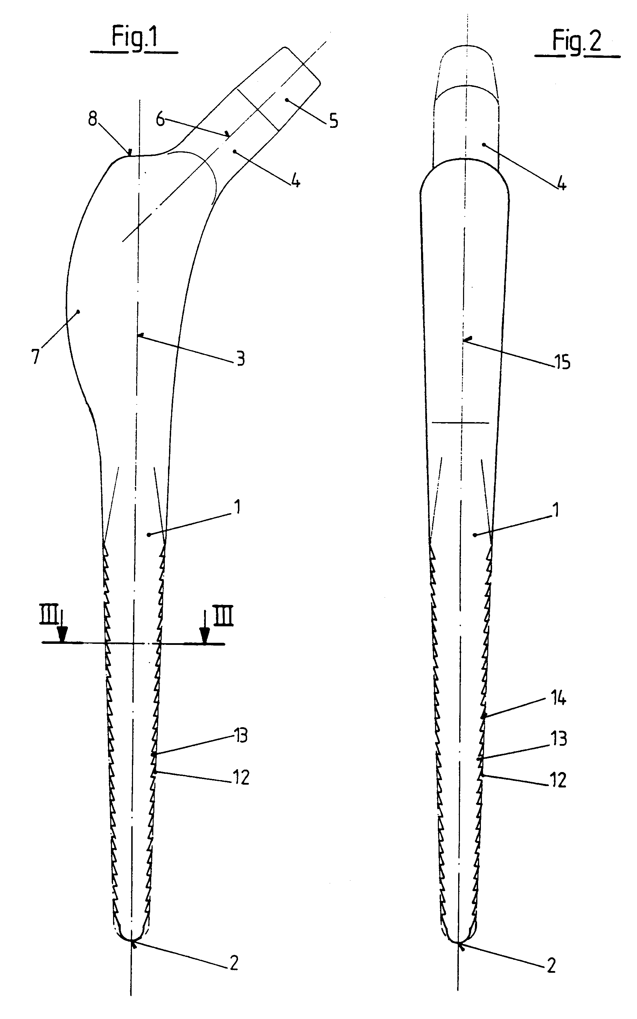

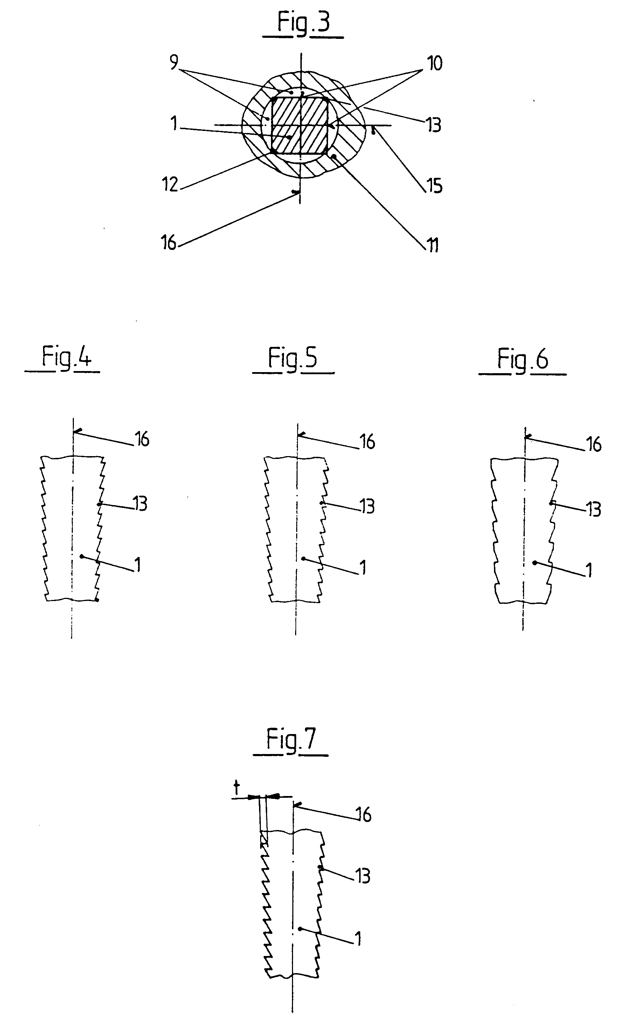

Wie Fig. 3 zeigt, ist der Querschnitt des Schaftes 1 im distalen Bereich quadratisch, wobei die Seiten 10 des Quadrates gerade, d.h. ohne Vertiefungen, ausgeführt sind. Der quadratische Querschnitt reicht dabei vom distalen Ende 2 bis etwa zur Hälfte der zwischen den beiden Enden 2 und 8 gemessenen Schafthöhe und geht dann in eine an den Ecken stark abgerundete rechteckige oder eine ovale Form über. Relativ zur durch die beiden Achsen 3 und 6 definierten Mittelebene 15 (Fig. 2) des Schaftes 1 ist der quadratische Querschnitt so orientiert, dass seine Seiten 10 Parallel und senkrecht zu dieser Ebene verlaufen. In einem zylindrisch konischen Operationshohlraum entstehen so Zwischenräume 9 zwischen dem kortikalen Knochen 11 und dem Schaft 1, in denen sich einerseits spongiöses Knochengewebe bildet und andererseits eine Revaskularisierung des Gewebes erfolgt.

Die eine Abstützung des Schaftes 1 auf dem kortikalen Knochen 11 bewirkenden Ecken 12 des Quadrates sind dabei mit Zähnen 13 besetzt, die sägezahnartig ausgebildet sind und - in dem Beispiel nach Fig. 1 bis 3 - alle mit ihren steilen Flanken 14 nach proximal weisen. Denn, um einen guten Halt gegen die auf der lateralen Seite bei Belastung auftretenden Zugkräfte zu gewährleisten, ist es notwendig, dass mindestens die Zähne 13, die lateral einer zweiten Mittelebene 16 liegen, die ihrerseits senkrecht auf der vorstehend definierten, ersten Mittelebene 15 steht, mit ihren steilen Flanken 14 nach proximal gerichtet sind. Die Zahntiefe t dieser lateral gelegenen Zähne 13 soll dabei grösser sein als diejenige der nach medial liegenden, wie dies in Fig. 7 wiedergegeben ist.

Beim Eindringen in das Knochengewebe üben Zähne 13, deren steile Flanken nach proximal weisen, eher eine das Gewebe verdrängende als eine schneidende Wirkung aus.

Unter Umständen ist medial im Gegensatz dazu eine schneidende Wirkung der Zähne 13 erwünscht; es ist daher möglich, die medial der zweiten Mittelebene 16 gelegenen Zähne 13 "umzudrehen", so dass die steilen Zahnflanken 14 nach distal weisen, was in Fig. 5 und ebenfalls in Fig. 7 gezeigt ist.

Weiterhin sind bei dem Beispiel nach den Fig. 1 bis 3 die Zähne 13 in Zahnkränzen angeordnet und folgen unmittelbar aufeinander. Selbstverständlich ist es jedoch auch möglich, statt der Zahnkränze die einzelnen Zähne 13 versetzt zueinander anzuordnen, wie dies in Fig. 4 gezeigt ist. Darüberhinaus kann man zwischen den einzelnen Zähnen 13 oder den Zahnkränzen Abstände vorsehen, in denen der ursprüngliche quadratische Querschnitt erhalten bleibt, was in Fig. 6 dargestellt ist.

## Patentansprüche

1. Geradschaft für eine Hüftgelenkprothese zur Verankerung im Femur (11), wobei der Schaft einen Längsabschnitt mit mit Sägezähnen (13) versehenen Anlageflächen (12) besitzt, welche im Querschnitt gesehen an den Ecken eines Vielecks angeordnet sind, wobei der Schaft (1) sich als Vollschaft vom distalen Ende (2) aus in Richtung seiner Längsmittelachse (3) allseitig konisch erweitert und wobei die steilen Zahnflanken (14) mindestens der Zähne (13) nach proximal weisen, die lateral derjenigen Mittelebene (16) des Schaftes (1) liegen, die senkrecht zur von Schaftlängsachse (3) und Prothesenhalsachse (6) gebildeten Ebene (15) verläuft, dadurch gekennzeichnet, daß der Schaft (1) mindestens im distalen Bereich einen quadratischen Querschnitt mit ebenen Seiten (10) bildet, und dass ferner ausschließlich im distalen Bereich die Ecken (12) des Quadrates mit den in axialer Richtung verteilten Zähnen (13) versehen sind.

2. Geradschaft nach Anspruch 1, dadurch gekennzeichnet, dass die Zahntiefe (t) der lateral der Mittelebene (16) gelegenen Zähne (13) grösser ist als diejenigen der medial davon liegenden.

3. Geradschaft nach Anspruch 1 oder 2, dadurch gekennzeichnet, dass die Zähne (13) in einzelnen Zahnkränzen angeordnet sind.

4. Geradschaft nach einem der Ansprüche 1 bis 2, dadurch gekennzeichnet, dass die Seiten (10) des Quadrates parallel und senkrecht zur Ebene von Längsmittelachse (3) und Prothesenhalsachse (6) verlaufen.

5. Geradschaft nach einem der Ansprüche 1 bis 3, dadurch gekennzeichnet, dass die steilen Zahnflanken (14) der medial der Mittelebene 16 gelegenen Zähne (13) nach distal gerichtet sind.

## Claims

1. A straight shaft for a hip joint prosthesis to be secured in the femur (11), whereby the shaft possesses a longitudinal portion having bearing surfaces (12) provided with saw teeth (13), which seen in cross section are disposed at the corners of a polygon, whereby the shaft (1) is conically widened on all sides as a complete shaft from the distal end (2) in the direction of its longitudinal central axis (3) and whereby the steep tooth faces (14) of at least the teeth (13), which lie laterally with respect to the median plane (16) of the shaft (1), which is perpendicular to the plane (15) formed by the longitudinal axis (3) of the shaft and the axis (6) of the prosthesis neck, point in the proximal direction,
**characterised in that** the shaft (1) forms a square cross section with plane sides (10) at least in the distal region,
**and in that,** exclusively in the distal region, the corners (12) of the square are also provided with the teeth (13) distributed in the axial direction.

2. A straight shaft according to claim 1,
**characterised in that** the tooth depth (t) of the teeth (13) located laterally with respect to the median plane (16) is greater than that of the teeth positioned medially with respect to said plane.

3. A straight shaft according to claim 1 or 2,
**characterised in that** the teeth (13) are disposed in individual toothed rings.

4. A straight shaft according to one of Claims 1 or 2,
**characterised in that** the sides (10) of the square extend parallel and perpendicular to the plane of the longitudinal central axis (3) and the prosthesis neck axis (6).

5. A straight shaft according to one of Claims 1 to 3,
**characterised in that** the steep tooth faces (14) of the teeth (13) positioned medially with respect to the median plane 16 are directed in the distal direction.

## Revendications

1. Tige rectiligne pour une prothèse d'articulation de la hanche à ancrer dans le fémur (11), la tige possèdant une partie longitudinale avec surfaces d'appui (12) pourvues de dents de scie (13), qui, vues en coupe transversale, se situent aux angles d'un polygone, dans laquelle la tige (1) s'élargit coniquement de tous côtés sous forme d'une tige massive, depuis l'extrémité distale (2) en direction de son axe médian longitudinal (3) et dans laquelle les flancs (14) raides des dents sont dirigés vers l'extrémité proximale, du moins pour ceux des dents (13) qui se situent sur le côté du plan médian (16) de la tige (1) qui s'étend perpendiculairement au plan (15) forme par l'axe longitudinal (3) de la tige et par l'axe du col de prothèse (6), caractérisée en ce que la tige (1) forme au moins dans la zone distale une section transversale carrée avec des côtés (10) plans et en ce qu'en outre les angles (12) du carré sont pourvus, exclusivement dans la zone distale, des dents (13) réparties dans la direction axiale.

2. Tige rectiligne selon la revendication 1, caractérisée en ce que la profondeur (t) des dents (13) situées sur le côté du plan médian (16) est supérieure à celle des dents situées en position médiale par rapport à celui-ci.

3. Tige rectiligne selon la revendication 1 ou 2, caractérisée en ce que les dents (13) sont disposées en couronnes dentées individuelles.

4. Tige rectiligne selon l'une des revendications 1 à 2, caractérisée en ce que les côtés (10) du carré sont parallèles et perpendiculaires au plan de l'axe médian longitudinal (3) et de l'axe du col de prothèse (6).

5. Tige rectiligne selon l'une des revendications 1 à 3, caractérisée en ce que les flancs (14) raides des dents (13) situées en position médiale par rapport au plan médian (16) sont orientés vers l'extrémité distale.
